# EUROPEAN PATENT SPECIFICATION

(11) **EP 1 292 316 B1**
(45) Date of publication and mention of the grant of the patent: **18.03.2009**
(21) Application number: 01928913.1
(22) Date of filing: 26.04.2001
(51) Int. Cl.: A61L 31/00, A61L 31/04, C08L 1/26, C08L 71/02, A61K 31/74, A61K 47/34, A61K 31/715, A61K 31/717, A61K 31/721, A61K 31/722, A61K 31/727, A61K 31/728, A61K 47/00, A61K 47/30, A61K 47/36

(54) **HEMOSTATIC COMPOSITIONS OF POLYACIDS AND POLYALKYLENE OXIDES AND METHODS FOR THEIR USE**
HAEMOSTATISCHE ZUSAMMENSTELLUNG VON POLYSÄUREN UND POLYALKYLENOXYDEN UND DEREN VERWENDUNGSMETHODEN
COMPOSITIONS HEMOSTATIQUES DE POLYACIDES ET D'OXYDES DE POLYALKYLENE, ET PROCEDES D'UTILISATION

(30) Priority: 28.04.2000 US 200457 P; 28.04.2000 US 200637 P
(43) Date of publication of application: 19.03.2003
(73) Proprietor: Fziomed Inc., San Luis Obispo, CA 93401 (US)
(72) Inventor: CORTESE, Stephanie, M., Atascadero, CA 93422 (US); SCHWARTZ, Herbert, E., Redwood City, CA 94062 (US); OPPELT, William, G., Arroyo Grande, CA 93420 (US)
(74) Representative: Turner, Craig Robert
(86) International application number: PCT/US2001/013520
(87) International publication number: WO 2001/082937

(56) References cited:
- WO-A-00/59516
- WO-A-98/57678
- US-A- 5 512 329
- US-A- 5 858 746
- US-A- 5 906 997
- US-A- 5 944 754
- US-A- 5 968 542
- US-A- 6 017 301
- US-A- 6 034 140
- US-A- 6 054 122
- US-A- 6 096 309

## Description

### RELATED CASES

This application claims priority under 35 U.S.C. § 120 to United States Provisional Patent Application Serial No: 60/200,457, filed April 28, 2000, United States Provisional Patent Application Serial No: 60/200,637, filed April 28, 2000, and to U.S. Utility Patent Application Serial No: 09/472,110, filed December 27, 1999, all patent applications herein incorporated fully by reference. This application is also related to United States Utility Patent Application titled "Polyacid/Polyalkylene Oxide Foams and Gels and Methods for Their Delivery", mark E. Miller, Stephanie M. Cortese, Herbert E. Schwartz, and William G. Oppelt, inventors, Attorney docket No: FZIO 6604 US0 SRM/DBB, filed concurrently, incorporated herein fully by reference.

### FIELD OF THE INVENTION

This invention relates generally to the delivery and use of polyacid/polyether complexes, cross-linked gels comprising polyacids, polyalkylene oxides and multivalent ions, the use of those compositions and gels to inhibit the formation of adhesions between tissues and to promote hemostasis.

### BACKGROUND OF THE INVENTION

Adhesions are unwanted tissue growths occurring between layers of adjacent bodily tissue or between tissues and internal organs. Adhesions commonly form during the healing which follows surgical procedures, and when present, adhesions can prevent the normal motions of those tissues and organs with respect to their neighboring structures.

Bleeding at a site of surgery or a wound can contribute to adhesion formation. Adherence of platelets and/or fibrin clots can promote scarring and the formation of fibrous tissue or undesired adhesions between tissues. Thus, it can be important to reduce post-surgical bleeding by providing hemostasis. Additionally, it can be important to prevent fibrin clots from forming on adjacent tissues (antithrombogenesis). Antithrombogenicity and hemostasis are not the same phenomena. Antithrombogenicity is a property of a surface to inhibit the adherence and/or activation of platelets on that surface. Hemostasis is a complex set of physiological events within blood vessels that ultimately can result in the cessation of blood flow due to hemorrhage. Antithrombogenicity can be an important part ofhemostasis, in that often an early event in hemostasis includes the adherence of platelets to a cut tissue, with subsequent clot formation at that site. Once a clot forms, it can occlude the opening in the blood vessel, thereby decreasing leakage of blood out of the blood vessel. Although formation of clots (thrombi) within and immediately around an injured blood vessel is often desirable, if bleeding extends to the surrounding tissues, clot formation at those more remote sites can be harmful and does not necessarily contribute to hemostasis.

The medical and scientific communities have studied ways ofreducing the formation of post-surgical adhesions by the use of high molecular weight carboxyl-containing biopolymers. These biopolymers can form hydrated gels which act as physical barriers to separate tissues from each other during healing, so that adhesions between normally adjacent structures do not form. After healing has substantially completed, the barrier is no longer needed, and should be eliminated from the body to permit more normal function of the affected tissues.

Several di fferent types of biopolymers have been used for this purpose. For example, Balazs et al., U.S. Pat. No. 4,141,973 discloses the use of a hyaluronic acid (HA) fraction for the prevention of adhesions. However, because HA is relatively soluble and readily degraded *in vivo,* it has a relatively short half-life in vivo of 1 to 3 days, which limits its efficacy as an adhesion preventative.

Methyl cellulose and methyl cellulose derivatives are also known to reduce the formation of adhesions and scarring that may develop following surgery. (Thomas E. Elkins, et al., Adhesion Prevention by Solutions of Sodium Carboxymethylcellulose in the Rat, Part I, Fertility and Sterility, Vol. 41, No. 6, June 1984; Thomas E. Elkins, M.D. et al., Adhesion Prevention by Solutions of Sodium Carboxymethylcellulose in the Rat, Part II, Fertility and Sterility, Vol. 41. No. 6, June 1984. However, these solutions are rapidly reabsorbed by the body and disappear from the surgical site.

Additionally, solutions of polyethers can also decrease the incidence of post-surgical adhesions. Pennell et al., U.S. Patent No. 4,993,585 describes the use of polyethylene oxide in solutions of up to 15% to decrease formation of post-surgical adhesions. Pennell et al., U.S. Patent No. 5,156,839 describes the use of mixtures of carboxymethylcellulose up to about 2.5 % by weight, and polyethylene oxide, in concentrations of up to about 0.5% by weight in physiologically acceptable, pH neutral mixtures. Because of the neutral pH, these materials do not form association complexes, and thus, being soluble, are cleared from the body within a short period of time.

Although certain carboxypolysaccharide-containing membranes have been described, prior membranes can have disadvantages for use to prevent adhesions under certain conditions. Butler, U.S. Patent No. 3,064,313 describes the manufacture of films made of 100% carboxymethylcellulose (CMC) with a degree of substitution of 0.5 and below, made insoluble by acidifying the solution to pH of between 3 and 5, and then drying the mixture at 70°C to create a film. These films were not designed to be used as anti-adhesion barriers.

Anderson, U.S. Patent No. 3,328,259 describes making films of water soluble cellulose compounds, alkali metal salts, and a plasticizing agent for use as external bandages. These materials are rapidly soluble in plasma and water and thus would have a very short residence time as an intact film. Therefore, these compositions are not suitable for alleviating surgical adhesions.

Smith et al., U.S. Patent No. 3,387,061 describes insoluble association complexes of carboxymethylcellulose and polyethylene oxide made by lowering the pH to below 3.5 and preferably below 3.0, and then drying and baking the resulting precipitate (see Example XXXVIII). These membranes were not designed for surgical use to alleviate adhesions. Such membranes are too insoluble, too stiff, and swell to little to be ideal for preventing post-surgical adhesions.

Burns et al., U.S. Patent No. 5,017,229 describes water insoluble films made of hyaluronic acid, carboxymethyl cellulose, and a chemical cross-linking agent. Because of the covalent cross-linking with a carbodiimide, these films need extensive cleaning procedures to get rid of the excess cross-linking agent; and because they are made without a plasticizer, they are too stiff and brittle to be ideally suited for preventing adhesions -- they do not readily conform to the shapes of tissues and organs of the body.

Thus, there is a need for anti adhesion membranes and gels that can be used under a variety of different circumstances. D. Wiseman reviews the state of the art of the field in Polymers for the Prevention of Surgical Adhesions, In: Polymeric Site-specific Pharmacotherapy, A.J. Domb, Ed., Wiley & Sons, (1994). A currently available antiadhesion gel is made of ionically cross-linked hyaluronic acid. (Huang et al., U.S. Pat. No. 5,532,221, incorporated herein fully by reference).

Ionic cross-linking of polysaccharides is well documented in the chemical and patent literature (Morris and Norton, Polysaccharide Aggregation in Solutions and Gels, Ch. 19, in Aggregation Processes in Solution, Wyn-Jones, E. and Gormally, J, Eds., Elsevier Sci. Publ. Co. NY (1983)). Each type of metal ion can be used to form gels of different polymers under specific conditions of pH, ionic strength, ion concentration and concentrations of polymeric components. For example, alginate (a linear 1,4 - linked beta-D-mannuronic acid, alpha-L-glucuronic acid polysaccharide) can form association structures between polyglucuronate sequences in which divalent calcium ions can bind, leading to ordered structures and gel formation. Similar calcium binding ability is also demonstrated by pectin which has a poly-D-galacturonate sequence. The order of selectivity of cations for pectins is Ba²⁺>Sr⁺>Ca²⁺. CMC also can bind to monovalent and divalent cations, and CMC solutions can gel with the addition of certain trivalent cations (Cellulose Gum, Hercules, Inc., page 23 (1984)).

Sayce et al. (U.S. Pat. No. 3,969,290) discloses an air freshener gel comprising CMC and trivalent cations such as chromium or aluminum.

Smith (U.S. Pat. No. 3,757,786) describes synthetic surgical sutures made from water-insoluble metal salts of cellulose ethers.

Shimizu et al. (U.S. Pt. No. 4,024,073) describe hydrogels consisting of water-soluble polymers such as dextran and starch chelated with cystine or lysine through polyvalent cations.

Mason et al. (U.S. Pat. No. 4,121,719) disclose CMC- and gum arabic-aluminum hydrogels used as phosphate binding agents in the treatment of hyperphosphatemia.

U.S. Pat. No. 5,266,326 describes alginate gels made insoluble by calcium chloride. An antiadhesion gel is made of ionically cross-linked hyaluronic acid (Huang et al., U.S. Pat. No. 5,532,221). Cross-linking is created by the inclusion of polyvalent cations, such as ferric, aluminum or chromium salts.

Therefore, the prior art discloses no membranes or gels which are ideally suited to the variety of surgical uses of the instant invention.

Pennell et al (U.S. Pat. No. 5,156,839) describes CMC solutions containing small amounts of high molecular weight PEO. In one embodiment, Pennell describes covalently cross-linking gels using dimethylolurea.

Schwartz et al (U.S. Pat: No.s 5,906,997, 6,017,301, and 6,034,140) describe membranes, hydrogels and association complexes of carboxypolysaccharides and polyethers for use as antiadhesion compositions. Because of the presence of polyethers in membranes made using these materials, these compositions exhibited certain antithrombogenic properties, including decreased platelet adhesion, decreased platelet activation, and decreased binding of fibrin and blood clots to membranes. U.S. Patent Application Serial No: 09/472,110, incorporated herein fully by reference, disclosed that multivalent cations including Fe³⁺, Al³⁺, and Ca²⁺, and/or polycations including polylysine and polyarginine can be used to provide intermolecular attraction, thereby providing a means of controlling viscoelastic properties of gels.

### SUMMARY OF THE INVENTION

Membranes, gels and foams based on association complexation between polyacids ("PA") and hydrophilic polyalkylene oxides ("PO") can exhibit both hemostatic and antithrombogenic properties. In certain embodiments, the materials can have different hemostatic properties depending upon the pH and the PA and PO contents of the compositions. The PA of this invention can be made with polyacrylic acid, carboxypolysaccharides such as CMC, and other polyacids known in the art. Ionically and non-ionically cross-linked gels of this invention can be made by mixing polyacid and polyether together, either in dry form or in aqueous solution, and adding a solution containing cations to provide cross-linking between the PA, the PO and the cations. The cations can be either H+ or multivalent cations including divalent and trivalent cations. The pH of the compositions can be adjusted to provide a desired degree of hemostatic effect. In certain embodiments, more acidic compositions can provide increased hemostatis. The membranes, gels and foams can then be sterilized and stored before use.

The invention relates to the use of a biocompatible association complex of a polyacid (PA) and a polyalkylene oxide (PO), which is hemostatic and antithiombogenic, in the manufacture of a composition for providing hemostatis, wherein the pH of said composition is below 7. 5, provided that the association complex is not a freeze dried sponge obtainable by:
(a) mixing 9.5 g of dry, powdered carboxymethylcellulose with a degree of substitution of 0.82 with 0.5 g of dry, powdered polyethylene oxide with a molecular weight of 8,000 d;
(b) adding the thus obtained mixture to 500 ml of deionized water and 3.2 ml of either (i) a 25.2% w/v solution of FeCl₂.6H₂O, (ii) a 22.5% w/v solution of AlCl₃.6H₂O or (iii) a 20.6% w/v solution of CaCl₂.2H₂O;
(c) adjusting the osmolality to a physiologically acceptable value of about 300 mmol/kg by adding about 13 ml of a 30% w/v NaCl solution and mixing;
(d) if necessary, adding 1.7N NHOH to the thus obtain gel so that it has a pH of 7.0; and
(e) freeze drying the gel to form an ion-associated freeze dried sponge.

One aspect of the invention is the use of a composition comprising an intermacromolecular association of a carboxypolysaccharide (CPS) and a polyether (PE), and, for example, a polyethylene glycol ("PEG") which exhibits both adhesion properties as well as hemostatic properties.

Another aspect of the invention comprises the use of foams from complexes of PA and PO.

Another aspect of this invention includes the use of PA/PO compositions which can be delivered as a spray, or can be dried into a sponge and delivered to a tissue.

The compositions of this invention can be used to inhibit post-surgical adhesions, to decrease the consequences of arthritis, and/or to provide a lubricant for numerous medical and/or veterinary uses.

Additionally, in accordance with some aspects of the invention, drugs can be included in the membranes or gels to deliver pharmacological compounds directly to - the tissues. Certain of these embodiments can include the use of thrombin or other hemostatic agents to inhibit bleeding at a surgical or wound site.

In certain embodiment, the compositions used can be sterilized using thermal methods, gamma irradiation, and ion beams which can alter the physical and other properties of the components. Alternatively, in other embodiments of this invention, the materials can be filter sterilized.

The materials are biocompatible, and are cleared from the body within a desired period of time, which can be controlled.

By using both gel compositions and membrane compositions together in the same treatment procedure, improved anti-adhesion properties can be achieved.

### DETAILED DESCRIPTION DEFINITIONS

Before describing the invention in detail, the following terms are defined as used herein.

The term "adhesion" means abnormal attachments between tissues and organs that form after an inflammatory stimulus such as surgical trauma.

The terms "adhesion prevention" and "anti-adhesion" means preventing or inhibiting the formation of post-surgical scar and fibrous bands between traumatized tissues, and between traumatized and nontraumatized tissues.

The term "antithrombogenic" means decreased adherence of platelets, decreased platelet activation, decreased fibrin adherence, and/or decreased blood clot adherence to the anti-adhesion composition.

The term "association complex" or "intermacromolecular complex" means the molecular network formed between polymers containing CPS, polyacids, PE, polyalkylene oxide and/or multivalent ions, wherein the network is cross-linked through hydrogen and/or ionic bonds.

The term "bioadhesive" means being capable of adhering to living tissue.

The term "bioresorbable" means being capable of being reabsorbed and eliminated from the body.

The term biocompatible" means being physiologically acceptable to a living tissue and organism.

The term "carboxymethylcellulose" ("CMC") means a polymer composed of repeating carboxylated cellobiose units, further composed of two anhydroglucose units (β-glucopyranose residues), joined by 1,4 glucosidic linkages. The cellobiose units are variably carboxylated.

The term "carboxypolysaccharide" ("CPS") means a polymer composed of repeating units of one or more monosaccharides, and wherein at least one of the monosaccharide units has a hydroxyl residue substituted with a carboxyl residue.

The term "chemical gel" means a gel network comprised of covalently cross-linked polymers.

The term "degree of substitution" ("d.s.") means the average number of carboxyl or other anionic residues present per mole of cellobiose or other polymer.

The term "discectomy" means a surgical operation whereby a ruptured vertebral disc is removed.

The term "endoscope" means a fiber optic device for close observation of tissues within the body, such as a laparoscope or arthroscope.

The term "fibrous tissue" means a scar or adhesions.

The term "foam" means a gel having bubbles of a foaming gas.

The term "gel pH" means the pH of the gel or the pH of the casting solution from which the gel or a partially dried form of the gel is formed.

The term "hemostasis" means cessation of bleeding from a surgical or trauma site.

The term "hemostatic agent" means a drug or chemical that promotes hemostasis.

The term "hyaluronic acid" ("HA") means an anionic polysaccharide composed of repeat disaccharide units of N-acetylglucosamine and glucuronic acid. HA is a natural component of the extracellular matrix in connective tissue.

The term "hydration" (also "swelling") means the process of taking up solvent by a polymer solution.

The term "hydrogel" means a three-dimensional network of hydrophilic polymers in which a large amount of water is present.

The term "laminectomy" means a surgical procedure wherein one or more vertebral lamina are removed.

The term "mesothelium" means the epithelium lining the pleural, pericardial and peritoneal cavities.

The term "peritoneum" means the serous membrane lining the abdominal cavity and surrounding the viscera.

The terms "physical gel," "physical network" and "pseudo gel" mean non-covalently cross-linked polymer networks wherein the association of polymers in these gels is characterized by relatively weak and potentially reversible chain-chain interactions, which can be comprised ofhydrogen bonding, ionic association, ionic bonding, hydrophobic interaction, cross-linking by crystalline segments, and/or solvent complexation.

The term "polyacid" ("PA") means molecules comprising subunits having dissociable acidic groups.

The term "polyalkylene oxide" ("PO") means non-ionic polymers comprising alkylene oxide monomers. Examples of polyalkylene oxides include polyethylene oxide (PEO), polypropylene oxide (PPO) and polyethylene glycol (PEG), or block copolymers comprising PO and/or PPO.

The term "polycation" means a polymer containing multiple positively charged moieties. Examples of polycations include polylysine, polyarginine, and chitosan.

The term "polyethylene glycol" ("PEG") means a non-ionic polyether polymer being composed of ethylene oxide monomers, and having a molecular weight in the range of about 200 daltons ("d") to about 5000 daltons.

The term "polyethylene oxide" ("PEO") means the non-ionic polyether polymer composed of ethylene oxide monomers The molecular weight of PEO as used herein is between 5,000 d and 8,000 kilodaltons ("kd").

The term "solids" used with reference to polymer compositions means the total polymer content as a weight percentage of the total weight of the composition.

The term "solids ratio" means the percentage of the total dry polymer contents as a weight percentage of the total solids content.

The term "tissue ischemia" means deprivation of blood flow to living tissues.

### DETAILED DESCRIPTION OF THE INVENTION

The present invention is directed to the use of compositions promoting hemostatis, reducing the formation of adhesions during and following surgery and/or wound healing by delivering to a wound or a tissue, an implantable, hemostatic, bioresorbable association complex of carboxypolysaccharides (CPS) or other polyacid (PA), a polyalkylene oxide (PO), such as a polyether (PE), a polyethylene glycol (PEG), and/or multivalent ions and/or polycations, Complexes in membrane form can generally be made by mixing appropriate amounts and compositions of CPS and PE together in solution, then, adjusting the pH to provide a desired degree of hemostasis. Gels and foams can be used either at neutral pH, slightly alkaline, or at acidic pH.

To form foams, the hydrogel or association complex can be charged with a gas at increased pressure. Upon releasing the pressure, the dissolved gas expands to create the foam. The foam is applied to the surgical site, and adheres to the tissues which, during wound healing, would otherwise tend to form adhesions between them. Some of the gas escapes from the foam and the foam returns to a more gel-like state. The complex remains at the site for different periods of time, defending upon its composition, method of manufacture, and upon post-manufacture conditioning. When the tissues have substantially healed, the complex then degrades and/or dissolves and is cleared from the body.

A possible mechanism for formation of cross-linked gels and foams of this invention is discussed in U.S. Patent 5,906,997, incorporated herein fully by reference. This possible mechanism involves the formation of hydrogen bonds between PA and PO moieties in solution. Further, adding multivalent cations can form additional, ionic bonding between the PA, PO and cations. These possible mechanisms are for illustration only, and are not intended to be limiting. Other mechanisms may be responsible for the effects of the compositions used in this invention.

### Compositions of Hemostatic Membranes, Gels and Foams

The carboxypolysaccharide, polyether and other components of the compositions used in this invention may be of any biocompativle sort, including but not limited to those described in US Patent 5,906,997 and US Patent Application Serial No. 09/472,110.

The pH of the compositions used in the present invention may be below about 7, between 1 and 7, alternatively between 2 and 7, in other embodiments, between 2.5 and 7, in other embodiments, between 3 and 7, and in yet other embodiments, between 3.5 and 6.0. For certain uses, a pH of about 4.1 is desired where there is a desirable balance between the bioadhesiveness, hemostasis, antiadhesion properties, the rates of bioresorbability and the biocompatability for several uses contemplated in the present invention.

Like other polymers which are known to swell when exposed to water, PA/PO gels and foams are also bioadhesive. A possible reason for this -phenomenon is that with increased hydration, more charges on the polyacid become exposed, and therefore may be made available to bind to tissue proteins. However, excessive hydration is detrimental to bioadhesion. Thus, a means of controlling the bioadhesiveness of membranes is to control their hydration properties.

In addition to decreasing the pH of the association complex, increased intermacromolecular association can be achieved using caboxylated PAs, such as CPSs, with increased degree of carboxyl substitution. By increasing the density of protonatable carboxyl residues on the CPS, there is increasing likelihood of hydrogen bond formation even at a relatively high pH. The degree of substitution of CPS must be greater than 0, i.e., there must be some carboxyl residues available for hydrogen bond formation. However, the upper limit is theoretically 3 for cellulose derivatives, wherein for each mole of the saccharide, 3 moles of carboxyl residues may exist. Thus, in the broadest application of the invention involving CPS as the polyacid, the d.s. is greater than 0 and up to and including 3. In other embodiments, the d.s. is between 0.3 and 2. CPS with d.s. between 0.5 and 1.7 work well, and CPSs with a d.s. of about 0.65-1.45 work well and are commercially available.

The complexes used in the instant invention are intended to have a finite residence time in the body. Once placed at a surgical or wound site, or site of inflammation, the foam is designed to serve as a hemostatic barrier for a limited time period. Once healing has substantially taken place, the anti-adhesion barrier naturally disintegrates, and the components are cleared from the body. The time taken to clear the body for certain embodiments is desirable no more than 29 days because of increased regulation by the Food and Drug Administration of devices intended to remain within the body for more than 30 days. However, it can be desirable to provide longer-duration compositions for certain long-term uses.

The mechanism for bioresorption of PA/PO complexes are not well understood. However, an early step in the process of bioresorption is solubilization of the network of polyacid and polyalkylene oxide. For example, when soluble, CMC and PEO can diffuse into the circulation and be carried to the liver and kidneys, where they may be metabolized or otherwise eliminated from the body. Additionally, enzymatic action can degrade carbohydrates. It is possible that enzymes contained in neutrophils and other inflammatory cells may degrade the polymer networks and thereby increase the rate of elimination of the components from the body.

The degradation and rate of solubilization and disruption of the membrane is manipulated by careful adjustment of the pH during formation of the association complexes, by varying the CPS/PE ratio, and by selecting the appropriate degree of substitution of the CPS and molecular weights of the PE and CPS. Decreasing the molecular weight of CPS increases its solubility. The strength of the membrane can be tailored to the surgical application. For example, certain surgical applications (e. g., spine ortendon) may require a stronger, more durable membrane than others (such as intraperitoneal applications). Manipulation of the above-mentioned experimental variables allows the manufacture and use of products with variable residence times in the body.

Biocompatability of CPS/PE complexes used in the present invention can be a function of its acidity. A highly acidic complex contributes a relatively larger total acid load to a tissue than does a more neutral complexe. Additionally, the more rapidly hydrogen ions dissociate from a complex, the more rapidly physiological mechanisms must compensate for the acid load by buffering, dilution and other mechanisms. To mimic the rate and total amount of acid given up by a membrane *in vivo,* membranes are placed in PBS solutions and the degree of acidification of the PBS is measured. In addition to membrane pH, membrane composition also influences the acid load delivered to the body. Moreover, by using a foam preparation, the total solids content of the antiadhesion dose can be less than for either non-foam gels or for membranes. Therefore, the total load of acid delivered to a tissue by an acidic foam can be reduced, decreasing any adverse effects of the composition's acidity.

### Ionically and Non-Ionically Cross-Linked Polyacid/Polyalkylene Oxide Gels and Foams

Other embodiments of the present invention are directed to the use of ionically and non-ionically cross-linked membranes, gels and foams for reducing surgical adhesions, decreasing the symptoms of arthritis, and providing biologically compatible lubricants. Methods for accomplishing these aims comprise the step of delivering to a wound or other biological site, an implantable, bioresorbable composition comprised of a polyacid and a polyether. The components of the composition can be associated with each other by way of hydrogen bonding, ionic bonding, ionic association or ionic cross-linking, although other mechanisms may be responsible for the association.

Certain embodiments having relatively little intermolecular ionic bonding can be more readily resorbed than embodiments having more bonding. Thus, increasing intermolecular bonding can increase residence time of the composition in the body, and therefore can remain at the site for a longer period of time than compositions having smaller degrees of intermolecular bonding. By way of example, by selecting compositions which provide the highest viscosity (see below), the residence time can be adjusted to provide a desired lifetime of antiadhesion effect. Additionally, in certain other embodiments, the compositions used in the instant invention can be dried to form a membrane, which can further increase the residence time at a tissue site. Thus, by selecting the chemical composition of the gel, and by selecting the form of the composition (e.g., gel or membrane) used, a desired combination of properties can be achieved to suit particular needs.

### Gel Structures

The gels used in this invention are termed "physical gels". The term physical gels has been used (de Gennes, P.G. Scaling Concepts in Polymer Physics. Ithaca, NY. Cornell University Press, pp.133, (1979)) to describe non-covalently cross-linked polymer networks. Physical gels are distinguished from "chemical gels" which are covalently cross-linked. Physical gels are relatively weak and have potentially reversible chain-chain interactions which may be comprised of hydrogen bonds, ionic association, hydrophobic interaction, stereo-complex formation, cross-linking by crystalline segments, and/or solvent complexation.

Non-ionically and ionically cross-linked gels can be made by mixing appropriate amounts and compositions of polyacids, polyether and optionally, cross-linking cations together in a solution. To form non-ionically associated compositions, the solution can be acidified to promote cross-linking of the polyacid and polyether molecules through hydrogen bonds as described for carboxypolysaccharides and polyethers above and in U.S. Patent No: 5,906,997; U.S. Patent No: 6,017,301; U.S. Patent No.: 6,034,140; U.S. Patent Application No.: 09/252,147, filed February 18, 1999, and U S. Patent Application No: 09/472,110, filed december 27, 1999. Each aforementioned Patent and Application is herein incorporated fully by reference.

Membranes or films can be made by pouring a solution of PA and PO, with or without multivalent cations onto a suitable flat surface, such as a tray, and permitting the mixture to dry to form a membrane at either reduced (>0.01 Torr) or normal (about 760 Torr) atmospheric pressure. The membranes, films or gels can be placed between tissues which, during wound healing, would form adhesions between them. The complex can remain at the site for different periods of time, depending upon its composition, method of manufacture, and upon post-manufacture conditioning. When the tissues have substantially healed, the complex can then degrade and/or dissolve and is cleared from the body.

Gels and membranes used in accordance with the invention can be made with desired degrees of viscosity, rigidity, different rates of bioresorbability, different degrees of bioadhesion, different degrees of anti-adhesion effectiveness and different degrees of hemostatic and antithrombogenic properties.

Compositions of PA and PO require only that the solutions of PA and PO can be handled easily. Dilute solutions (up to about 10% weight/volume) of CPS are easy to handle, and solutions of about 2% CPS are easier to handle. Solutions of PEO up to about 20% (weight/volume) are possible to make and handle, and solutions of about 1% by weight are easy to handle. However, the maximal concentration can be increased if the molecular weight of the PE is reduced. By way of example only, PEG having a molecular weight of about 1000 Daltons can be made in a concentration of about 50%. Further decreasing the molecular weight of the PE can permit even higher concentrations to be made and handled easily.

### B. Polyacid Components

The polyacid may be of any biocompatible sort. By way of example, a group of polyacids useful for the present hemostatic invention are carboxypolysaccharides (CPS) including carboxymethyl cellulose (CMC), carboxyethyl cellulose, chitin, carboxymethyl chitin, hyaluronic acid, alginate, pectin, carboxymethyl dextran, carboxymethyl chitosan, and glycosaminoglycans such as heparin, heparin sulfate, and chondroitin sulfate. Additionally, polyuronic acids such as polymannuronic acid, polyglucuronic acid, and polyguluronic acid, as well as propylene glycol alginate can be used. In addition to the CPS, polyacrylic acids, polyamino acids, polylactic acid, polyglycolic acids, polymethacrylic acid, polyterephthalic acid, polyhydroxybutyric acid, polyphosphoric acid, polystyrenesulfonic acid, and other biocompatible polyacids known in the art are suitable for making foams. Such polyacids are described in Biodegradable Hydrogels for Drug Delivery, Park et al., Ed., Technomic Publishing Company, Basel, Switzerland (1993), incorporated herein fully by reference. Preferably, carboxymethylcellulose or carboxyethylcellulose is used. More preferably, carboxymethylcellulose (CMC) is used. The molecular weight of the carboxypolysaccharide can vary from 10 kd to 10,000 kd. CPS in the range of from 600 kd to 1000 kd work well, and CPS of 700 kd works well, and is easily obtained commercially.

### C. Polyalkylene Oxide Components

Similarly, many polyalkylene oxides can be used. These include polypropylene oxide (PPO), PEG, and PEO and block co-polymers of PEO and PPO, such as the Pluronics™(a trademark of BASF Corporation, North Mount Olive, New Jersey). A preferred PO of the present invention is polyethylene oxide (PEO) having molecular weights of between about 5,000 Daltons (d) and about 8,000 Kd. Additionally, polyethylene glycols (PEG) having molecular weights between about 200 d and about 5 kd are useful.

The inclusion of a polyether in the complex confers antithrombogenic properties which help prevent adhesions by decreasing the adherence of blood proteins and platelets to a composition (M. Amiji, Biomaterials, 16:593-599 (1995); Merill, E.W., PEO and Blood Contact in Polyethylene Glycol Chemistry-Biotechnical and Biomedical Applications, Harris J. M. (ed), Plenum Press, NY, 1992; Chaikofet al., A.I. Ch.E. Journal 36(7):994-1002 (1990)). PEO-containing compositions impair the access of fibrin clots to tissue surfaces, even more so than a composition containing CMC alone. The inclusion of PE to the gels also can increase the spreading or coating ability of the gel onto biological tissues. By increasing the spreading, there is increased likelihood that the gel can more efficiently coat more of the tissue and thereby can decrease the likelihood of formation of adhesions at sites remote from the injured tissue.

Varying the ratios and concentrations of the polyacid, the polyether and multivalent cations or polycations can alter hemostatic and antithrombogenic properties. In general, increasing the amount of CPS and decreasing the amount of PO can increase hemostasis, whereas increasing the amount of PO an decreasing the amount of CPS can decrease hemostasis.

The percentage ratio of PA to PO may be from about 10% to 99% by weight, alternatively between about 50% and about 99%, and in another embodiment about 90% to about 99%. Conversely, when the PO is PE, the percentage of PE can be from about 1% to about 90%, alternatively from about 1% to about 50%, and in another embodiment, about 1% to 10%. In another embodiment, the amount of PE can be about 2.5%.

### D. Ionic Components

The tightness of the association and thus the physical properties of ionically associated PA/PO compositions used in the instant invention may be closely regulated by selection of appropriate multivalent cations. In certain embodiments, it can be desirable to use cations selected from different groups of the periodic table. Increasing the concentration and/or valence of polyvalent cations can increase ionic bonding. Therefore, trivalent ions of the periodic table such as Fe³⁺, Al³⁺, Cr³⁺ can provide Stronger ionic cross-linked association complexes than divalent ions such as Ca²⁺, Mg⁺⁺, Mn⁺⁺ or Zn²⁺. However, other cations can be used to cross-link the polymers of the gels used in this invention. Polycations such as polylysine, polyarginine, chitosan, or any other biocompatible, polymer containing net positive charges under aqueous conditions can be used. The anions accompanying the cations can be of any biocompatible ion. Typically, chloride (Cl) can be used, but also PO₄²⁻, HPO₃⁻ , CO₃²⁻, HCO₃⁻ , SO₄²⁻, borates such as B₄O₇²⁻ and many common anions can be used. Additionally, certain organic polyanions can be used. By way of example, citrate, oxalate and acetate can be used. In certain embodiments, it can be desirable to use hydrated ion complexes, because certain hydrated ion salts can be more easily dissolved that anhydrous salts.

Moreover, in non-ionically associated PA/PO complexes, hydrogen bonding may be a mechanism for associating the polymers together. According to one hypothesis, decreasing the pH of the association complex can increase the amount of hydrogen bonding between PA and PO components. Similarly, increasing the degree of substitution of the carboxypolysaccharide in the gel can increase cross-linking within the association complex at any given pH or ion concentration. The pH of the membranes and gels used can be below about 7.5, alternatively between about 2 and about 7.5, alternatively between about 6 and about 7.5, and in other embodiments, about 3.5 to about 6.

Moreover, we unexpectedly found that decreasing the pH of the composition can increase hemostatic effect. Thus, the hemostatic compositions used can have pH in the range of below about 7.0, alternatively, below about 6.0, in other embodiments below about 5.0, in yet further embodiments below about 4.0, and in still other embodiments, below about 3.0.

Membranes and gels having high solids %, or high degrees of cross-linking, such as those made using trivalent cations in the concentration range providing maximal ionic association can dissolve more slowly than gels made with lower ion concentration and/or with ions having lower valence numbers. Such membranes and gels can be used advantageously during recovery from surgery to ligaments and tendons, tissues which characteristically heal slowly. Thus, a long-lasting composition could minimize the formation of adhesions between those tissues.

### III. Incorporation of Drugs into Compositions

Ionically cross-linked and non-ionically cross-linked gels and membranes can be made which incorporate drugs to be delivered to the surgical site. Incorporation of drugs into membranes is described in Schiraldi et al., U.S.Patent No. 4,713,243 and in U.S. Patent 5,906,997, incorporated herein fully by reference. The incorporation of drugs into the compositions used in the instant invention may be either the manufacturing stage or added later but prior to insertion. Drugs which may inhibit adhesion formation include antithrombogenic agents such as heparin or tissue plasminogen activatory, drugs which are anti-inflammatory, such as aspirin, ibuprofen, ketoprofen, or other, non-steroidal anti-inflammatory drugs. Furthermore, hormones, cytokines, osteogenic factoris, chemotactic factors, proteins and peptides that contain an arginine-glycine-aspartate ("RGD") motif, analgesics or anesthetics may be added to the compositions, either during manufacturer or during conditioning. Any drug or other agent which is compatible with the compositions and methods of manufacture maybe used with the present invention. Desirably, to increase hemostatic properties of gels and foams, hemostatic agents, including vasoconstrictors, fibrillar collagen and clotting factors such as thrombin can be added. Vasoconstrictors can include adrenergic agonists, for example, norepinephrine, epinephrine, phenylpropanolamine, dopamine, metaraminol, methoxamine, ephedrine, and propylhexedrine.

### IV. Uses of PA/PO Compositions

The types of surgery in which the gel and/or foam compositions of the instant invention may be used is not limited. Examples of surgical procedures are described in U.S. Patent Nos: 5,906,997, 6,017,3401, and 6,034,140 as well as U.S. Patent Application No: 09/472,110, filed December 27, 1999, each patent and application incorporated herein fully by reference. Additionally, wound healing can be augmented for a variety of wounds, including abdominal injury, muscular injuries, skin injuries, and other soft-tissue injuries. Moreover, in certain embodiments, the gels of this invention can be placed at a desired site using an endoscope. Such types of administration can include laparoscopy, endoscopy and injection through needles.

### V. Polyacid/Polyalkylene Oxide Foams and Delivery Systems for Gels and Foams

In other embodiments of this invention, foams of polyacids and polyalkylene oxides are used. Foams offer advantages over gels in that they can require less material, the material can be less dense, and therefore can be applied more easily against a gravity gradient, i.e., uphill, and can adhere more evenly to a tissue without flowing or sliding off. To make PA/PO foams, typically a mixture of PA/PO gel is exposed to increased pressure in the presence of a charging gas, including but not limited to CO₂, N₂, a noble gas such as helium, neon, argon, or any other gas that is relatively inert physiologically and does not adversely affect the polyacid or polyalkylene oxide or other components of the mixture.

The gel material can be loaded into a pressurized canister, such as those used for aerosol applications. Upon releasing the pressure, such as by opening the valve, the pressure in the canister forces some of the gas/gel mixture out of the canister, thereby relieving the pressure on the gel. Some gas dissolved in the gel comes out of solution and can form bubbles in the gel, thereby forming the foam. The foam then expands until the gas pressure within the foam reaches equilibrium with the ambient pressure. In some embodiments, the bubbles can coalesce and can ultimately disperse, leaving the mixture in a gel-like state, adhering to the tissue.

In certain other embodiments, it can be desirable to include a surface-active agent in the mixture to prolong the time that the foam remains in the foamy state. Any surfactant can be used that is biocompatible and does not adversely affect the materials in the foam.

Delivery systems for gels and foams are further described in the concurrently filed Utility Patent Application titled "Polyacid/Polyalkylene Foams and Gels and Methods for Their Delivery" Mark E. Miller, Stephanie M. Cortese, Herbert E. Schwartz and William G. Oppelt, inventors. The above patent application is herein incorporated by reference in its entirety.

In general, delivery systems for gels comprise the composition to be delivered, a pressurized container and a valve. The composition is loaded into the canister under pressure, and when a valve is opened, the composition flows out of the canister under pressure. In certain embodiments, hemostatic antithrombogenic compositions can be delivered to a surgical site using such delivery systems.

The hemostatic compositions used in the mutant invention can also be used in sponge form. Manufacture of sponges is described in US Patent Application No. 09/472,110, incorporated herein fully by reference.

### VI. Examples

In the following examples, PA/PO gel compositions are described for CMC as an exemplary carboxypolysaccharide, and PRO is the exemplary polyalkylene oxide. It is understood that association complexes of other carboxypolysaccharides, other polyacids, polyethers and other polyalkylene oxides can be made and used in similar ways. Thus, the invention is not limited to the use of these Examples, but can be practiced in any equivalent fashion without departing from the invention.

### Example 1: Antithrombogenic effect of CMC/PEO Membranes I

Samples of CMC (7 HF PH) and CMC/PEO (5000 kd) membranes were made with CMC/PEO ratios of 80%/20%, 65%/35%, and 50%/50% at a pH of from 2.7 to 2.9. An observation chamber for adherent platelets was assembled consisting of a polymer-coated glass slide, two polyethylene spacers, and a glass coverslip. Human blood, obtained from healthy adult volunteers after informed consent, was collected in heparin-containing evacuated containers (Vacutainer^{™}, Becton-Dickinson, Rutherford, NJ). Heparinized blood was centrifuged at 100g for 10 mm to obtain platelet-rich plasma (PRP).

Two hundred microliters ("µL") of PRP was instilled into the platelet observation chamber. Platelets in PRP were allowed to adhere and activate on the polymer surfaces for 1 hr at room temperature. Non-adherent platelets and plasma proteins were removed by washing the chamber with PBS. Adherent platelets were fixed with 2.0% (w/v) glutaraldehyde solution in PBS for 1 hour After washing with PBS, the platelets were stained with 0.1% (w/v) Coomassie Brilliant Blue (Bio-Rad, Hercules, CA) dye solution for 1.5 hours. Stained platelets were observed using a Nikon Labophot^{™} II light microscope at 40X magnification (Melville NY). The image of adherent platelets was transferred to a Sony Trinitron^{™} video display using a Mamamatsu CCD^{™} camera (Mamamatsu-City, Japan). The Hamamatsu Argus- 10^{™} image processor was used to calculate the number of platelets per 25,000 µm² surface area in every field of observation. The extent of platelet activation was determined qualitatively from the spreading behavior of adherent platelets. Images of activated platelets were obtained from the Sony Trinitron^{™} video display screen using a Polaroid ScreenShooter^{™} camera (Cambridge, MA).

The number of adherent platelets and the extent of platelet activation are considered early indicators of the thrombogenicity of blood-contacting biomaterials. Platelet activation was measured qualitatively by the extent of platelet spreading on the polymer surfaces. The extent of platelet spreading was judged from 1 (least reactive) to 5 (most reactive) as described in Table 1, which is based on the criteria of Lin et al., Polyethylene surface sulfonation. Surface characterization and platelet adhesion studies. J. Coll. Interface Sci. 164: 99-106 (1994), incorporated herein fully by reference.

**Table 1**

| **Evaluation of Platelet Activation: Surface-Induced Spreading** | | |
|---|---|---|
| **Platelet Activation Stage** | **Approximate Spread Area (µm²)** | **Remarks** |
| 1 | 10 - 15 | Contact-adherence. Platelets not active. |
| 2 | 15 - 25 | Partially active. Initiation of pseudopods. |
| 3 | 25 - 35 | Partially activated. Pseudopod extension and initiation of release of granular contents. |
| 4 | 35 - 45 | Partially activated. Significant pseudopod formation and extension. Complete release of granular contents. |
| 5 | > 45 | Fully activated. Retraction of pseudopods leading to the flat or "pancake" shape. |

**Table 2**

| **Platelet Adherence And Activation By CMC/PEO Membranes** | | |
|---|---|---|
| **Membrane Composition** | **Number of Adherent Platelets (per 25,000 µm²)^{a}** | **Extent of Activation (µm²)** |
| **100% CMC** | 95.8±15.3 | 2.96±0.37 |
| **80% CMC/20% PEO** | 48.1 ± 10.9 | 3.25 ±0.35 |
| **65% CMC/35% PEO** | 17.8 ±4.25 | 1.57 ±0.39 |
| **50% CMC/50% PEO** | 5.25 ±2.67 | 1.00 ±0.00 |
| **a: mean ± standard deviation (n=24).** | | |

Table 2 shows that significant number of platelets had adhered and activated on membranes made of 100% CMC. On the average, more than 95 activated platelets were present per 25,000 µm². The number of adherent platelets and the extent of activation decreased with increasing PEO content in the membranes. The membranes having a CMC/PEO ration of 50%/50% had the least number of platelets. On the average, only 5 contact-adherent platelets were present on these membranes.

The results of this study indicate that CMC/PEO membranes, especially the 50%/50% CMC/PEO membrane, is highly anti-thrombogenic, based on the reduction in the number of adherent platelets and the extent of platelet activation on these surfaces. Thus, increasing the amount of PEO in membranes increases their antithrombogenic properties.

### Example 2: Blood Prothrombin Time after Spinal Injection of CMC/PEO Mixtures

To determine whether CMC and PEO adversely affect blood clotting *in vivo,* we performed a series of studies in which we injected CMC/PEO mixtures into the spines of rabbits, and measured prothrombin time in blood drawn from the animals.

Four rabbits (2.4 to 2.8 kg) were anesthetized using ketamine (40 mg/kg) and xylazine (8 mg/kg), and 0.20 ml of clinical grade 2% CMC, 0.05% PEO, 50% H₂0 and 47.9% balanced salt solution (Lot #SD011089) was injected into the lower spinal area using a 27-gauge, ½ inch needle. A fifth, uninjected rabbit (2.8 kg) served as the control. Blood samples (approximately 1.6 ml) were taken at 0 (before injection), 2, 6, 24, 48, and 96 hr post dose. To 1.6 ml of the collected blood, 0.2 ml of 3.8% sodium citrate solution was added. After mixing plasma was prepared by centrifuging the sample at 2000 rpm for 3 to 5 minutes in a clinical centrifuge. Plasma was pipetted into a separate labeled tube and kept on ice. The sample was frozen and sent to California Veterinary Diagnostics, Inc., West Sacramento, CA for prothrombin-time determination, which was conducted in compliance with FDA's Good Laboratory Practice Regulations.

Table 3 shows the prothrombin times for each sample of rabbit plasma at various sampling times. Rabbit blood coagulates more quickly than human blood (Didisheim et al., J. Lab. Clin. Med. 53, 866-1959); thus, several of the samples collected from these rabbits coagulated before analysis. However, the samples assayed showed no effect of the CMC/PEO mixture on the prothrombin time except for rabbit No. 3, which showed a transient increase but recovered by day 4. We conclude that dural application of CMC/PEO mixtures do not adversely affect whole blood prothrombin time.

**Table 3**

| **Prothrombin Time (Seconds) of Rabbits Injected with CMC/PEO** | | | | | |
|---|---|---|---|---|---|
| | **Rabbit Number** | | | | |
| **Time (hr)** | **1** | **2** | **3** | **4** | **5*** |
| **0** | 7.2 | 7.2 | 7.1 | 8.4 | 7.1 |
| **2** | - | 7.1 | 7.1 | 7.1 | 7.1 |
| **6** | 7.3 | 7.1 | 7.1 | 7.8 | 7.1 |
| **24** | 7.2 | 7.i | 10.6 | 7.1 | 8.0 |
| **48** | 7.3 | - | 10.3 | - | - |
| **96** | 6.2 | 6.5 | 6.5 | 6.0 | 6.0 |
| ***Control rabbit not injected with CMC/PEO.** | | | | | |
| **- indicates that assay was not performed because the sample had coagulated.** | | | | | |

### Example 3: Surface and Blood-Contacting Properties of CMC/PEO Films Introduction:

The purpose of this study was to determine whether the CMC/PEO membranes of this invention have anti-thrombogenic properties. CMC (700 kd) and PEO (4400 kd) were blended and the mixture was cast into thin films at a pH of 4.2. The bilayered films had approximately the same thickness as the monolayered films. Also, for the bilayered films, the different layers had about the same mass. The films were evaluated for surface and blood compatibility properties.

### A. Platelet Adhesion and Activation II:

### Introduction

Platelet adhesion and activation is an important indicator of blood-biomaterial interactions (Hoffman. Blood-Biomaterial Interactions: An Overview. In S.L. Copper and N.A. Peppas (eds). Biomaterials: Interfacial Phenomena and Applications. Volume 199. American Chemical Society, Washington, DC. 1982 pp 3-8, incorporated herein fully by reference). The initial number of adherent platelets and the extent of platelet activation on biomaterial surface correlates with the potential long-term blood-compatibility profile (Baier et al. Human Platelet Spreading on Substrata of Known Surface Chemistry. J. Biomed. Mater. Res. 19: 1157-1167 (1985), incorporated herein fully by reference). When in contact with polymeric surfaces, platelets initially retain their discoid shape present in the resting state and the spread area is typically between 10 - 15 µm². Upon activation, platelets extend their pseudopods and initiate the release of granular contents. During the partial activation stage, the area of the spread platelet can increase to about 35 µm². When the platelets are fully-activated, they retract the pseudopods to form circular or "pancake" shape and the spread area increases to 45 or 50 µm² (Park et al. Morphological Characterization of Surface-Induced Platelet Activation. Biomaterials 11: 24-31 (1990), incorporated herein fully by reference). The spreading profiles of activated platelets were used to create five activation stages as described by Lin *et al.* (Lin et al. Polyethylene Surface Sulfonation: Surface Characterization and Platelet Adhesion Studies. J. Coll. Interface. Sci. 164: 99-106 (1994), incorporated herein fully by reference). Clean glass promotes platelet adhesion and activation (Park et al. The Minimum Surface Fibrinogen Concentration Necessary for Platelet Activation on Dimethyldichlorosilane-Coated Glass. J. Biomed. Mater. Res. 25: 407-420 (1991), incorporated herein fully by reference).

### Methods

Platelet adhesion and activation measurement was performed as previously described (M. Amiji, Permeability and Blood Compatibility Properties of Chitosan-Poly(ethylene oxide) Blend Membranes for Hemodialysis. Biomaterials 16: 593-599 (1995), M. Amiji. Surface Modification of Chitosan Membranes by Complexation-Interpenetration of Anionic Polysaccharides for Improved Blood Compatibility in Hemodialysis. J. Biomat. Sci., Polym. Edn. 8: 281-298 (1996), both articles incorporated herein fully by reference). Briefly, a platelet observation chamber was assembled consisting of film-covered clean glass slide, two polyethylene spacers, and a glass coverslip. Human blood, obtained from healthy adult volunteers after informed consent, was collected in heparin-containing evacuated containers (Vacutainers®, Becton-Dickinson, Rutherford, NJ). Heparinized blood was centrifuged at 100g for 10 minutes to obtain platelet-rich plasma (PRP).

The polymer compositions studied included a non-irradiated film A having side 1 composed of 95% CMC and 5% PEO, and side 2 composed of 60 % CMC and 40% PEO. Film B was otherwise identical to Film A, except that the film had been irradiated with γ-radiation as described in U.S. Application No: 09/472,110, incorporated herein fully by reference. Films C and D were made of 77.5% CMC and 22.5 % PEO and film C was not irradiated, whereas film D was irradiated. Film E was 100% CMC and was irradiated.

To measure platelet adherence and activation, two-hundred (200) µL of PRP was instilled into the platelet observation chamber. Platelets in PRP were allowed to adhere and activate on the polymer surfaces for one hour at room temperature. Non-adherent platelets and plasma proteins were removed by washing the chamber with phosphate-buffered saline (PBS, pH 7.4). Adherent platelets were fixed with 2.0% (w/v) glutaraldehyde solution in PBS for 1 h. After washing with PBS, the platelets were stained with 0.1 % (w/v) Coomassie Brilliant Blue (Bio-Rad, Hercules, CA) dye solution for 1.5 h. Stained platelets were observed using a Nikon Labophot® II (Melville, NY) light microscope at 40X magnification. The image of adherent platelets was transferred to a Sony Trinitron® video display using a Hamamatsu CCD® camera (Hamamatsu-City, Japan). The Hamamatsu Argus-10® image processor was used to calculate the number of platelets per 25,000 µm² surface area in every field of observation. The data indicates average number of adherent platelets ± S.D. from at least twelve fields of observation and two independent experiments.

The extent of platelet activation was determined qualitatively from the spreading behavior of adherent platelets as described above in Table 4.

### Results:

The extent of platelet adhesion was determined by counting the number of platelets per 25,000 µm² surface area. Surface-induced platelet activation was measured qualitatively from the spreading behavior of adherent platelets as shown in Table 4.

**Table 4**

| **Platelet Adherence and Activation by Control and CMC/PEO Films^{a}** | | |
|---|---|---|
| **Film** | **Number of Platelets (per 25,000 µm²)** | **Extent of Activation (µm²)** |
| Glass | 1 57.3 ± 1 9.6^{b} | 4.8 ± 0.3 |
| A, side 1 | 26.0 ± 5.4 | 2.2 ± 0.1 |
| A, side 2 | 6.2 ± 2.2 | 1.2 ± 0.4 |
| B, side 1 | 27.9 ± 7.3 | 2.4 ± 0.3 |
| B, side 2 | 6.0 ± 2.9 | 1.2 ± 0.1 |
| C | 3.5 ± 1.7 | 1.0 ± 0.0 |
| D | 3.4 ± 1.1 | 1.0 ± 0.0 |
| E | 62.8 ± 12.4 | 3.6 ± 0.4 |

As shown in Table 4, platelets adhered to the glass surface and became activated. Platelets did not adhere in as great a number to CMC/PEO membranes, however, and were not activated to the same degree as by glass. The degree of adherence and activation was inversely related to the PEO concentration. Thus, increasing the amount of PEO decreased both platelet adherence and platelet activation. Moreover, comparing films A and C (radiated) with films B and D (non-radiated) there was no effect of gamma radiation on platelet adhesion and activation.

From the platelet adhesion and activation studies, increased surface PEO correlated with reduced adherence and activation of platelets. Based on these observations, CMC-PEO membranes with high PEO content are relatively non-thrombogenic.

### B. Plasma Recalcification Time:

### Introduction

Plasma recalcification time measures the length of time required for fibrin clot formation in calcium-containing citrated plasma that is in contact with the surface of interest. It is a useful marker of the intrinsic coagulation reaction. Plasma recalcification time is a measure of the intrinsic coagulation mechanism (Renaud, The recalcification plasma clotting time. A valuable general clotting test in man and rats. Can. J. Physiol. Pharmacol. 47: 689-693 (1969), incorporated herein fully by reference). Since the time required for contact activation of plasma varies with the type of surface, the plasma recalcification time is used as an indicator of blood compatibility of biomaterials (Rhodes et al., Plasma recalcification as a measure of the contact phase activation and heparinization efficacy after contact with biomaterials. Biomaterials 15: 35-37 (1994), incorporated herein fully by reference).

### Methods

Human blood was collected in evacuated containers (Vacutainers, Becton-Dickinson) in the presence of sodium citrate buffer as an anticoagulant. Citrated blood was centrifuged at 2,500g for 20 minutes to obtain platelet-poor plasma. A round sections (20 mm in diameter) of the control and CMC-PEO films were cut with an aid of a sharp scalpel. Tissue Culture Polystyrene (TCP) surfaces are created by treating polystyrene microplates with oxygen plasma to convert the hydrophobic surface into a hydrophilic one. The film sections were placed in 12-well tissue-culture polystyrene (TCP, Falcon®, Becton-Dickinson) microplates and hydrated with 2.0 ml of PBS for 10 minutes: Excess PBS was removed by suction.

The compositions tested were the same as described above for platelet adhesion and activation. Film A had side 1 composed of 95% CMC and 5% PEO, and side 2 composed of 60 % CMC and 40% PEO. Film B was otherwise identical to Film A, except that the film had been irradiated with γ-radiation as described in U.S. Application No: 09/472,110, incorporated herein fully by reference. Films C and D were made of 77.5% CMC and 22.5 % PEO and film C was not irradiated, whereas film D was irradiated. Film E was 100% CMC and was irradiated.

Plasma recalcification time of citrated plasma in contact with control and CMC-PEO blend films was measured according to the procedure described by Brown (Brown, Hematology: Principles and Procedures. Sixth Edition. Lea and Febioger, Philadelphia, PA. 1993, pp. 218, incorporated herein fully by reference). Briefly, 1.0 ml of citrated plasma was mixed with 0.5 ml of 0.05 M calcium chloride and incubated with hydrated film samples in a water-bath at 30° C. The samples were occasionally removed from the water-bath and gently stirred. The time required for fibrin clot formation was recorded. The data indicates average of the plasma recalcification time ± S.D. from four independent experiments. Plasma recalcification time was determined using the methods of Renaud and Rhodes et al., cited above. The results of this study are presented in Table 5.

**Table 5**

| **Recalcification Time for Plasma in Contact with Control and CMC-PEO Films^{a}** | |
|---|---|
| **Film** | **Plasma Recalcification Time (minutes)** |
| Control TCP^{b} | 6.3 ± 0.2^{c} |
| A, side 1 | 13.9 ± 0.6 |
| A, side 2 | 17.8 ± 0.5 |
| B, side 1 | 13.5 ± 0.9 |
| B, side 2 | 17.8 ± 0.6 |
| C | 15.3 ± 0.8 |
| D | 15.1 ± 0.5 |
| E | 5.6 ± 0.3 |

| | |
|---|---|
| ^{a} The time required for fibrin clot formation with calcium-containing citrated human plasma was measured in minutes. ^{b} Tissue-culture polystyrene (TCP) 12-well microplate was used as a control. ^{c} Mean ± S.D. (n=4). | |

The contact activation time on TCP was about 6.3 minutes, and on 100% CMC (film E) was about 5.6 minutes. This is similar to the contact activation time previously found for clean glass surfaces. In contrast, the plasma recalcification times on PEO-containing films (samples A-D) were significantly higher than the control TCP or CMC surfaces. The recalcification time correlated with the increased PEO content of the film, with increased PEO resulting in increased recalcification time. Therefore, contact activation of plasma was substantially reduced for membranes with increased amounts of PEO.

### Conclusions:

Films containing increased amounts of PEO on their surfaces are anti-thrombogenic and can prevent formation of fibrin clots from forming on the surfaces of the films. The antithrombogenic effects are dependent on the amount of PEO. Thus, manufacturing films having increased PEO concentration can decrease thrombogenicity.

### Example 4: Hemostatic Effects of CMC/PEO Membranes

The purpose of these studies is to determine the hemostatic properties of CMC/PEO polymer preparations. These studies were carried out at Livingston Research Institute under the direction of the inventors.

### Introduction

Examples 1-3 above demonstrate some effects of CMC/PEO membranes to inhibit thrombogenesis, that is, the adherence and activation of platelets in blood. However, antithrombogenicity and hemostasis are not the same phenomena. Antithrombogenicity is a property of a surface to inhibit the adherence and/or activation of platelets on that surface. Hemostasis is a complex set of physiological events within blood vessels that ultimately can result in the cessation of blood flow due to hemorrhage. According to a possible mechanism of hemostasis, within seconds of a vascular trauma, platelets adhere to the subendothelial collagen exposed by the trauma. Once a monolayer of platelets is formed, mediators can be released from the adherent platelets, and those mediators can recruit additional platelets to aggregate upon the adherent platelets. This process can continue until a platelet "plug" is formed. The platelet plug can be stabilized by a fibrin network formed as a result of activation of the coagulation cascade. The platelet/fibrin plug can grow in size until the lumen of the hemorrhaging blood vessel is occluded and blood flow stops. Thus, an antithrombogenic property of a composition is not necessarily inconsistent with the hemostatic property of the composition. Hemostasis can also be promoted by constriction, or narrowing, of the local blood vessels.

### Methods:

Animals: Twenty-three (23) New Zealand White rabbits, 2.4 - 2.7 kg each, were purchased from Irish Farms (Norco, CA) and quarantined in the University of Southern California ("USC") vivarium for at least 2 days prior to use. Three rabbits were used for preliminary experiments. Twenty rabbits were divided into five treatment groups of four animals each, prior to initiation of surgery. The animals were housed with a light:dark cycle of 12 hrs:12 hrs, were fed *ad libitum.*

Animals were anesthetized using ketamine (55 mg/kg/xylazine (5 mg/kg), intramuscularly. The abdominal area was shaved and prepared for sterile surgery with Betadine and alcohol solution. A midline laparotomy was performed

Materials: The CMC/PEO polymer gels used had a total solids content of 2% in distilled water, the solids being 90% CMC (7HF, Hercules) and 10 % PEO (4.4 Md molecular weight). Gels were made according to methods in the U.S. Patent Application No: 09/472,110, filed December 27, 1999. For membrane studies, membranes were 77.5 % CMC (7HF)/22.5 % PEO (4.4 Md) at a pH of either 3.0 ("SPF 3.0") or 4.0 ("SPF 4.0"), made according to methods described in U.S. Application No: 09/472,110, filed December 27, 1999. When dried, the membranes had thicknesses of between about 0.0022" and about 0.0028".

### Splenic Injury

A 4 x 4 inch gauze sponge was used to isolate the spleen. A lacerating apparatus was made by clamping a No. 15 scalpel blade in a straight hemostat so that 2 mm of the cutting edge projected from the side of the hemostat. A uniform laceration was made by pulling the blade along the greater curvature of the spleen, beginning about 1 mm from the upper pole and ending about 1 mm from the lower pole.

### Hepatic Injury

The liver was exteriorized from the abdomen and gently laid on a gauze sponge. Hepatic injury was made using a metal template. A liver wound was made by pressing a metal template on the surface of the exteriorized liver and excising the protruding tissue with a sharp blade. The injured area was 3 cm².

### Application of Hemostatic CMC/PEO Compositions

After injury, the affected organ was treated by applying the hemostatic composition to the site. For the liver injuries, the hemostatic material was applied and gentle pressure was applied. Observations were made over an 18 minute period, and the total time, in minutes, required to achieve complete hemostasis was measured.

### Preliminary Studies

Three rabbits were used for preliminary studies. One animal received a splenic injury, one animal received a hepatic injury and one animal received both splenic and hepatic injuries. In the one animal in which both injuries were made, we found that one injury made it difficult to interpret the results of hemostasis at the other site. Thus, for the further experiments, we made only hepatic injuries to the animals.

### Results

The effects of CMC/PEO compositions on bleeding time (in minutes) are shown in Table 6.

**Table 6**

| **Effects of CMC/PEO Gels and Membranes on Bleeding Time in Rabbits^{a}** | | | | | | |
|---|---|---|---|---|---|---|
| **Animal No:** | **1** | **2** | **3** | **4** | **Mean** | **SEM** |
| **Control** | > 18 | 9.75 | 11.0 | > 18 | 14.18 | 1.92 |
| **Gelfoam^{™}** | 9.08 | 6.25 | 2.83 | 3.0 | 5.28 | 1.29 |
| **SPF-3** | 1.50 | 2.75 | 1.17 | 1.33 | 1.68 | 0.31 |
| **SPF-4** | 2.50 | 3.83 | 3.0 | 2.53 | 2.97 | 0.27 |
| **SPG** | 2.75 | 4.67 | 4.0 | 6.08 | 4.33 | 0.60 |

| | | | | | | |
|---|---|---|---|---|---|---|
| a: Dam expressed as mean ± standard error of the mean (SEM). | | | | | | |

The results show that the control animals had a long bleeding time (over 14 minutes). Each of the treated animals had decreased bleeding time. Unexpectedly, the animals receiving the membranes having a pH of 3 had the shortest bleeding time, being less than about 0.1 of the time of the control animals. The membrane having a pH of 4 was also effective, requiring about 1/5 the time to achieve hemostasis. The gel-treated animals showed a bleeding time of 4.33 minutes, which represents a decrease of about 70% compared to untreated control animals, and about 20 % compared to Gelfoam™-treated animals. Animals treated with Gelfoam^{™} also had reduced bleeding time compared to untreated control animals. In general, it appears that the membrane embodiments used in this invention have slightly greater hemostatic properties than Gelfoam^{™}, with bleeding times being about 1/3 and 2/3, respectively, for pH 3 and pH 4, of the bleeding time observed with Gelfoam^{™}.

It can be appreciated that with reduced pH, the acid load delivered to tissues can be increased compared to compositions having higher pH. In certain embodiments of hemostatic membranes, the membranes can be made thin. For example for acidic membranes having the same surface area and pH, a membrane having only one-half the thickness will deliver only about one-half the acid load to the tissue. Thus, by making acidic membranes very thin, the desired hemostatic property can be achieved while minimizing adverse effects of delivering a high acid load to the animal and tissue.

### Example 5: Polyacid/Polyalkylene Oxide Foams

In addition to the membranes and gels described other embodiments used in this invention include foams. Foams of PA/PO mixtures can be made by dissolving a gas, such as CO₂ or N₂ in the mixture under more than atmospheric pressure. The gas and mixture is allowed to equilibrate so that the partial pressure of the gas in the mixture is about the same as the partial pressure of the gas in the gas phase. Any device can be used to deliver foams comprising the compositions used in this invention. It can be desired to use a delivery system as described in the concurrently filed U.S. Utility Patent Application titled: "Polyacid/Polyether Foams and Gels and Methods for Their Delivery" Mark E. Miller, Stephanie M. Cortese, Herbert E. Schwartz and William G. Oppelt, inventors, filed concurrently. This patent application is incorporated herein fully by reference.

### Example 6: Hemostatic Comparison of CMC/PEO Gels

The purpose of this study was to evaluate the ability of CMC/PEO gels to perform as hemostatic agents in a common animal model of profuse hepatic bleeding. The study was performed under the inventors direction at Covance Research Laboratories.

### Introduction

Hemostatic evaluation of CMCD/PEO gels and film formulations used in this invention and a prior art product (Gelfoam^{™}) was carried out in an animal model of profuse bleeding at Livingston Research Institute. This study indicated that each of the gel and film formulations tested were successful in reducing the bleeding time.

In another study, CMC/PEO gel formulations exhibited hemostatic properties in a Lee-White blood clotting model. This in vitro method tested the ability of gel formulations, with and without added thrombin, to clot human blood. We compared the CMC/PEO preparations with Proceed^{™} (Fusion Medical). This study showed substantially decreased clotting time compared to controls. Gel preparations used in this invention with thrombin showed an even greater decrease in clotting time as compared to the controls, and was comparable to the clotting time observed for Proceed^{™}.

### Materials

Two types of CMC/PEO gels were used in this study. Both were composed of 90% CMC 10% PEO (dry weight percentages). The CMC was 7HF from Hercules and the PEO had a 4.4 Md molecular weight from RITA). However, Gel. A was made with 3.1 % total solids content, whereas Gel B had 3.4 % total solids content. The gels were made according to methods disclosed in U.S. Patent Application Serial No: 09/472,110, filed December 27, 1999, incorporated herein fully by reference.

Dry CMC and PEO were mixed before being added to a vortexing solution of deionized water (1500 ml), calcium chloride and sodium chloride. Once the dry chemicals were completely incorporated into the solution, the speed of vortexing was reduced and the gel was allowed to mix for approximately two hours to achieve homogeneity. The gel was then filtered into syringes and sterilized in a steam autoclave.

The osmolality was then adjusted to a physiologically acceptable value of about 300 mmol/kg by adding about 13 ml of a 30% w/v solution of NaCl and further mixing the gel. The calcium ion-associated gels did not require any pH adjustment after their manufacture. The gel was then sterilized in an autoclave for 15 minutes at 250° C.

### Methods

One adult pig was anesthetized. The domestic pig was used because its liver is sufficiently large to accommodate the required number of test sites. Following preparation for surgery, a midline incision as made to perform a laparatomy. The liver was exposed and surface defects were created using a template to guide in the preparation of a 1 cm x 1 cm surface defect to create profuse bleeding. The template was pressed onto the surface of the liver and the protruding tissue was first scored along the perimeter with a scalpel blade, pulled up on the center with tweezers, and then cut underneath to remove the one square cm flap so produced.

Once the injury was made, the injury site was patted with gauze to remove excess blood, the gel product was then applied, and tamponade was immediately applied using gauze for one minute. Control sites received the standard one minute tamponade without any gel preparation. After one minute, the injury site was observed to see if bleeding had stopped. If bleeding had stopped, the time was recorded, and if not, the site was allowed to compoete its clotting cycle without additional tamponade. In cases where bleeding was still very active at the one-minute time point, tamponade was applied at one-minute intervals. The recorded "clotting time" recorded was the time from the removal of the 1 cm x 1 cm flap of liver until the blood completely clotted. A standard volume (0.5 ml) of test gel was applied to each site followed by tamponade as described.

The total number of sites so created in one animal did not exceed 35 sites. There were 7 sites for each test material and 7 control sites available. As bleeding at each site stopped, another site was prepared and used to measure hemostasis with another gel sample.

### Results

The results follow in Table 7 and illustrate the hemostatic capability of the CMC/PEO gels used in this invention compared with that of Proceed^{™}.

**Table 7**

| **Effect of CMC/PEO Gels on Bleeding Time in Pig Hepatic Model** | | | |
|---|---|---|---|
| Test Article | Clotting Time (min) | Average | Standard Deviation |
| Gel A + thrombin | 1.35 | 1.65 | 0.30 |
| | 1.50 | | |
| | 1.72 | | |
| | 2.03 | | |
| Gel B + thrombin | 1.55 | 1.59 | 0.28 |
| | 1.42 | | |
| | 1.45 | | |
| | 2.08 | | |
| | 1.43 | | |
| Gel B alone | 9.23 | 10.38 | 2.75 |
| | 15.0 | | |
| | 10.0 | | |
| | 7.68 | | |
| | 10.0 | | |
| Proceed^{™} | 2.05 | 1.49 | 0.42 |
| | 1.53 | | |
| | 1.08 | | |
| | 1.28 | | |
| Blood only | 8.37 | 9.12 | 1.03 |
| | 10.0 | | |
| | 8.10 | | |
| | 10.0 | | |

### Conclusion

The results of the above studies demonstrated that the thrombin-containing CMC/PEO gels used in this invention are effective hemostatic agents. On average, gels of this invention having thrombin decreased clotting time to about 15% of the sites treated with gel without thrombin. Moreover, the gel having higher total solids content (Gel B) had a slightly better hemostatic effect than the gel (Gel A) having lower total solids content. Additionally, Gel B decreased clotting time to about 17% of the time needed for those sites not exposed to any hemostatic agent (untreated controls).

## Claims

1. Use of a biocompatible association complex of a polyacid (PA) and a polyalkylene oxide (PO), which is hemostatic and antithrombogenic, in the manufacture of a composition for providing hemostasis, wherein the pH of said compositions is below 7.5,
provided that the association complex is not a freeze dried sponge obtainable by:
(a) mixing 9.5 g of dry, powdered carboxymethylcellulose with a degree of substitution of 0.82 with 0.5 g of dry, powdered polyethylene oxide with a molecular weight of 8,000 d;
(b) adding the thus obtained mixture 10 500 ml of deionized water and 3,2 ml of either (i) a 25.2% w/v solution of FeCl₂-6H₂O, (ii) a 22:5% w/v solution of AlCl₃•6H₂O or (iii) a 20.6% w/v solution of CaCl₂•2H₂O
(c) adjusting the osmolality to a physiologically acceptable value of about 300 mmol/kg by adding about 13 ml of a 30% w/v NaCl solution and mixing;
(d) if necessary, adding 1:7N NHOH to the thus obtained gel so that it has a pH of 7.0; and
(e) freeze drying the gel to from an ion-associated freeze dried sponge.

2. Use according to claim 1, wherein the pH of said composition is below 6.0.

3. Use according to claim 1 or 2, wherein said polyacid is selected from a carboxypolysaccharide, polyacrylic acid, polyamino acid, polylactic acid, polyglycolic acid, polymethacrylic acid, polyterephthalic acid, polyhydroxybutyric acid, polyphosphoric acid, polystyrenesulfonic, acid, and copolymers of said polyacids.

4. Use according to claim 1 or 2, wherein the polyacid is a carboxypolysaccharide selected from carboxymethyl cellulose (CMC), carboxyethyl cellulose, chitin, carboxymethyl chitin, hyaluronic acid, alginate, propylene glycol alginate, pectin, carboxymethyl dextran, carboxymethyl chitosan, heparin, heparin sulfate, chondroitin sulfate and polyuronic acids including polymannuronic acid, polyglucuronic acid and polyguluronic acid.

5. Use according to any preceding claim wherein said polyalkylene oxide is selected from polypropylene oxide, polyethylene glycol, polyethylene oxide and PEO/PPO copolymers.

6. Use according to any preceding claim wherein said composition includes a multivalent cation.

7. Use according to claim 6, wherein said multivalent cation is selected from Ca²⁺, Mg²⁺, Mn²⁺, Fe³⁺, Cr³⁺, Zn²⁺ and Al³⁺.

8. Use according to claim 6 wherein said composition further comprises a divalent cation selected from Ca²⁺, Mg²⁺, Mn²⁺, and Zn²⁺.

9. Use according to any preceding claim wherein said composition further comprises a drug

10. Use according to claim 9 wherein said drug is selected from antithrombogenic drugs, hemostatic agents, anti-inflammatory drugs, hormones, chemotactic factors, analgesics, growth factors, cytokines, osteogenic factors and anesthetics.

11. Use according to claim 8 wherein said drug is selected from heparin, tissue plasminogen activator, thrombin, aspirin, ibuprofen, ketoprofen, proteins and peptides containing an RGD motif, and nonsteroidal anti-inflammatory drugs.

12. Use according to claim 9 wherein said drug is a hemostatic agent

13. Use according to claim 9 wherein said hemostatic agent is thrombin.

## Patentansprüche

1. Verwendung eines biokompatiblen Assoziationskomplexes von einer Polysäure (PA) und einem Polyalkylenoxid (PO), der blutstillend ist und antithrombogen wirkt, bei der Herstellung einer Zusammensetzung zur Bereitstellung von Hämostase, wobei der pH-Wert der Zusammensetzung unter 7,5 liegt,
vorausgesetzt, dass der Assoziationskomplex nicht ein gefriergetrockneter Schwamm ist, der folgendermaßen erhalten werden kann:
(a) Mischen von 9,5 g trockener, pulverförmiger Carboxymethylcellulose mit einem Substitutionsgrad von 0,82 mit 0,5 g trockenem, pulverförmigem Polyethylenoxid mit einer relativen Molekülmasse von 8000 d;
(b) Zugeben des so erhaltenen Gemischs zu 500 ml vollentsalztem Wasser und 3,2 ml von entweder (i) einer 25,2%-igen (w/v) Lösung von FeCl₂•6H₂O, (ii) einer 22,5%-igen (w/v) Lösung von AlCl₃•6H₂O oder (iii) einer 20,6%-igen (w/v) Lösung von CaCl₂•2H₂O;
(c) Einstellen der Osmolalität auf einen physiologisch annehmbaren Wert von etwa 300 mmol/kg, indem etwa 13 ml einer 30%-igen (w/v) NaCl-Lösung zugegeben werden und es gemischt wird;
(d) falls erforderlich, Zugeben von 1,7N NHOH zu dem so erhaltenen Gel, so dass es einen pH-Wert von 7,0 aufweist; und
(e) Gefriertrocknen des Gels, um einen ionenassoziierten gefriergetrockneten Schwamm zu bilden.

2. Verwendung nach Anspruch 1, wobei der pH-Wert der Zusammensetzung unter 6,0 liegt.

3. Verwendung nach Anspruch 1 oder 2, wobei die Polysäure aus einem Carboxypolysaccharid, Polyacrylsäure, Polyaminosäure, Polymilchsäure, Polyglykolsäure, Polymethacrylsäure, Polyterephthalsäure, Polyhydroxybuttersäure, Polyphosphorsäure, Polystyrolsulfonsäure und Copolymeren der genannten Polysäuren ausgewählt ist.

4. Verwendung nach Anspruch 1 oder 2, wobei es sich bei der Polysäure um ein Carboxypolysaccharid handelt, das aus Carboxymethylcellulose (CMC), Carboxyethylcellulose, Chitin, Carboxymethylchitin, Hyaluronsäure, Alginat, Propylenglykolalginat, Pektin, Carboxymethyldextran, Carboxymethylchitosan, Heparin, Heparinsulfat, Chondroitinsulfat und Polyuronsäuren, einschließlich Polymannuronsäure, Polyglucuronsäure und Polyguluronsäure, ausgewählt ist.

5. Verwendung nach einem der vorhergehenden Ansprüche, wobei das Polyalkylenoxid aus Polypropylenoxid, Polyethylenglykol, Polyethylenoxid und PEO/PPO-Copolymeren ausgewählt ist.

6. Verwendung nach einem der vorhergehenden Ansprüche, wobei die Zusammensetzung ein mehrwertiges Kation enthält.

7. Verwendung nach Anspruch 6, wobei das mehrwertige Kation aus Ca²⁺, Mg²⁺, Mn²⁺, Fe³⁺, Cr³⁺, Zn²⁺ und Al³⁺ ausgewählt ist.

8. Verwendung nach Anspruch 6, wobei die Zusammensetzung weiterhin ein zweiwertiges Kation umfasst, das aus Ca²⁺, Mg²⁺, Mn²⁺ und Zn²⁺ ausgewählt ist.

9. Verwendung nach einem der vorhergehenden Ansprüche, wobei die Zusammensetzung weiterhin ein Arzneimittel umfasst.

10. Verwendung nach Anspruch 9, wobei das Arzneimittel aus antithrombogen wirkenden Arzneimitteln, blutstillenden Mitteln, Antiphlogistika, Hormonen, chemotaktischen Faktoren, Analgetika, Wachstumsfaktoren, Zytokinen, osteogenen Faktoren und Anästhetika ausgewählt ist.

11. Verwendung nach Anspruch 8, wobei das Arzneimittel aus Heparin, Gewebsplasminogenaktivator, Thrombin, Aspirin, Ibuprofen, Ketoprofen, Proteinen und Peptiden, die ein RGD-Motiv enthalten, und nicht-steroidalen Antirheumatika ausgewählt ist.

12. Verwendung nach Anspruch 9, wobei es sich bei dem Arzneimittel um ein blutstillendes Mittel handelt.

13. Verwendung nach Anspruch 9, wobei es sich bei dem blutstillenden Mittel um Thrombin handelt.

## Revendications

1. Utilisation d'un complexe d'association biocompatible d'un polyacide (PA) et d'un oxyde de polyalkylène (PO), qui est hémostatique et antithrombogène, dans la fabrication d'une composition pour fournir une hémostase, dans laquelle le pH de ladite composition est inférieur à 7,5,
à la condition que le complexe d'association ne soit pas une éponge lyophilisée pouvant être obtenue par :
(a) mélange de 9,5 g de carboxyméthylcellulose en poudre sèche avec un degré de substitution de 0,82 avec 0,5 g d'oxyde de polyéthylène en poudre sec avec un poids moléculaire de 8,000 d ;
(b) ajout du mélange ainsi obtenu à 500 ml d'eau désionisée et 3,2 ml soit (i) d'une solution à 25,2 % poids/volume de FeCl₂-6H₂O, ou (ii) d'une solution à 22,5 % poids/volume d'AlCl₃•6H₂O, ou (iii) d'une solution à 20,6 % poids/volume de CaCl₂•2H₂O ;
(c) ajustement de l'osmolalité à une valeur physiologiquement acceptable d'environ 300 mmoles/kg en ajoutant environ 13 ml d'une solution de NaCl à 30 % poids/volume et en mélangeant ;
(d) si nécessaire, ajout de 1,7 N de NHOH au gel ainsi obtenu de sorte qu'il présente un pH de 7,0 ; et
(e) lyophilisation du gel pour former une éponge lyophilisée associée à des ions.

2. Utilisation selon la revendication 1, dans laquelle le pH de ladite composition est inférieur à 6,0.

3. Utilisation selon la revendication 1 ou la revendication 2, dans laquelle ledit polyacide est sélectionné parmi un carboxypolysaccharide, acide polyacrylique, acide polyamino, acide polyactique, acide polyglycolique, acide polyméthacrylique, acide polytéréphtalique, acide polyhydroxybutyrique, acide polyphosphorique, acide polystyrènesulfonique, et des copolymères desdits polyacides.

4. Utilisation selon la revendication 1 ou la revendication 2, dans laquelle le polyacide est un carboxypolysaccharide sélectionné parmi la carboxyméthylcellulose (CMC), carboxyéthylcellulose, chitine, carboxyméthylchitine, acide hyaluronique, aginate, aginate de propylèneglycol, pectine, carboxyméthyldextrane, carboxyméthylchitosane, héparine, sulfate d'héparine, sulfate de chondroïtine et acides polyuroniques y compris acide polymannuronique, acide polyglucuronique et acide polyguluronique.

5. Utilisation selon l'une quelconque des revendications précédentes, dans laquelle ledit oxyde de polyalkylène est sélectionné parmi de l'oxyde de polypropylène, du polyéthylèneglycol, de l'oxyde de polyéthylène et des copolymères PEO/PPO.

6. Utilisation selon l'une quelconque des revendications précédentes, dans laquelle ladite composition comprend un cation multivalent.

7. Utilisation selon la revendication 6, dans laquelle ledit cation multivalent est sélectionné parmi Ca²⁺, Mg²⁺, Mn²⁺, Fe³⁺, Cr³⁺, Zn²⁺ et Al³⁺.

8. Utilisation selon la revendication 6, dans laquelle ladite composition comprend en outre un cation divalent sélectionné parmi Ca²⁺, Mg²⁺, Mn²⁺ et Zn²⁺.

9. Utilisation selon l'une quelconque des revendications précédentes, dans laquelle ladite composition comprend en outre un médicament.

10. Utilisation selon la revendication 9, dans laquelle ledit médicament est sélectionné parmi des médicaments antithrombogènes, des agents hémostatiques, des médicaments anti-inflammatoires, des hormones, des facteurs chimiotactiques, des analgésiques, des facteurs de croissance, des cytokines, des facteurs ostéogènes et des anesthésiques.

11. Utilisation selon la revendication 8, dans laquelle ledit médicament est sélectionné parmi de l'héparine, un activateur du plasminogène des tissus, de la thrombine, de l'aspirine, de l'ibuprofène, du kétoprofène, des protéines et des peptides contenant un motif RGD, et des médicaments anti-inflammatoires non stéroïdiens.

12. Utilisation selon la revendication 9, dans laquelle ledit médicament est un agent homéostatique.

13. Utilisation selon la revendication 9, dans laquelle ledit agent homéostatique est la thrombine.
